Europäisches Patentamt

European Patent Office

Office européen des brevets

(11) Publication number: **0 248 144 B1**

(12) **EUROPEAN PATENT SPECIFICATION**

(45) Date of publication of patent specification: **21.10.92**   (51) Int. Cl.5: **G01N 33/573**, //G01N33/577, G01N33/543

(21) Application number: **87102122.6**

(22) Date of filing: **14.02.87**

(54) Selective immunoassay for pro-urokinase and urokinase.

(30) Priority: **27.02.86 GB 8604851**

(43) Date of publication of application:
**09.12.87 Bulletin 87/50**

(45) Publication of the grant of the patent:
**21.10.92 Bulletin 92/43**

(84) Designated Contracting States:
**AT BE CH DE ES FR GB GR IT LI LU NL SE**

(56) References cited:
**EP-A- 0 084 344**
**WO-A-86/01411**

**CHEMICAL ABSTRACTS, vol. 100, no. 13, 26 March 1984, Columbus, OH (US); G.SALERNO et al., p. 261, no. 98781m**

**CHEMICAL ABSTRACTS, vol. 99, no. 5, 01 August 1983, Columbus, OH (US); P.HERION et al., p. 225, no. 34891a**

**CHEMICAL ABSTRACTS, vol. 106, no. 1, 05 January 1987, Columbus, OH (US); M.L.NOLLI et al., p. 351, no. 3605u**

(73) Proprietor: **GRUPPO LEPETIT S.p.A.**
**Via G. Murat 23**
**I-20159 Milano(IT)**

(72) Inventor: **Corti, Angelo**
**2, Via Mozzi**
**I-24100 Bergamo(IT)**
Inventor: **Nolli, Marialuisa**
**15, Via Cavallini**
**I-27100 Pavia(IT)**
Inventor: **Cassani, Giovanni**
**3, Via Vittadini**
**I-27100 Pavia(IT)**
Inventor: **Parenti, Francesco**
**24, Via Benvenuto Cellini**
**I-20020 Lainate (Milano)(IT)**

(74) Representative: **Sgarbi, Renato et al**
**GRUPPO LEPETIT S.p.A. Patent and Trademark Department Via Roberto Lepetit, 34 I-21040 Gerenzano (Varese)(IT)**

**Description**

The present invention is directed to a method of selective assay of urinary plasminogen activator zymogen (pro-uPA) (commonly known also as pro-urokinase) and urinary plasminogen activator (u-PA) (commonly known also as urokinase) in biological fluids as well as in any kind of solution containing them, either separately or jointly.

One feature of this method is in fact that it allows the determination of pro-uPA also in the presence of its enzymatically active derivative u-PA.

The method of the invention is accurate, precise, rapid and easy to practice. The intra- and inter- assay precision are well within the range of values currently accepted for analytical purposes.

According to the method of the invention u-PA and/or pro-uPA are selectively immunoadsorbed on the surface of analytical means coated with a monoclonal antibody directed to an epitope which is common to pro-uPA and u-PA and the samples are tested for enzymatic activity with and without activation of the enzymatically inactive zymogen.

The accuracy of this assay is due also to the fact that the impurities and interfering materials are easily removed from the sample after immunoadsorption of the analyte(s).

Plasminogen activators (PA) are serine proteases which convert plasminogen into plasmin, a protease with high specificity for fibrin which is the main constituent of blood clots. At least two immunological distinct types of PAs have been isolated from cultured malignant cell lines: those related to the tissue plasminogen activator (TPA) (see Rijken D.C., Wijngaard G. Zaal-de Jong M., Welbergen J. (1979) Biochim. Biophys Acta 380, 140) and those related to the urinary activator (u-PA) (also called urokinase) (see Williams J.R.B. (1951) Br. J. Exp. Pathol. 32, 530). The u-PA type, originally purified as a two chain protein from urine, has been recently isolated in a proenzyme form (called pro-uPA) for instance, from a human epidermoid carcinoma cell line HEp3 (see Wun T.D., Ossowsky L. and Reich E. (1982) J. Biol. Chem. 257, 7262), from human glioblastoma cells (see Nielsen L.S. et al. (1982) Biochem 21, 6410), from urine (see Husain S.C. et al. (1983) Arch. Biochem. Biophys 220, 31) and from the human kidney permanent cell line TCL-598 (see Kohno T. et al. (1984) Biotechnol 2, 628). A proenzyme immunologically related to u-PA has been identified also from the supernatant of the human epidermoid carcinoma cell line A431.

This pro-uPA zymogen is a single chain protein of 50-54000 with no or low amidolytic activity and high fibrin affinity, it is resistant to diisopropylfluorophosphate treatment and inactivation by plasma inhibitors (see Gurewich V. et al. (1984) J. Clin. Invest. 73, 1731) and it is convertible into the two-chain active enzyme (u-PA) by treatment with plasmin.

The human u-PA molecule has been isolated in two biologically active forms: a high molecular weight form (MW about 54,000) and a low molecular weight form (MW about 33,000). The low molecular weight form (LMW) is derived from the high molecular weight form (HMW) by enzymatic cleavage.

The HMW form consists of a 30,000 heavy chain (B-chain) and a 20,000 light chain (A-chain) linked by a disulfide bond. The LMW form consists of the whole B-chain which contains the active site and a small proteolytically resistant fragment (MW = 2,427) derived from the A-chain linked by a disulfide bond. The A-chain is proteolytically splitted into a 18,000 fragment and the above 2,427 fragment. It seems that the LMW u-PA is less active than the HMW u-PA in accelerating clot lysis in vivo (M. Samama et al., Thromb. Haemostas. 40, 578-580, (1979) and G. Murano et al., Blood, 55, 430-436 (1980)). Human u-PA is at present mainly obtained from biological sources such as human urine, tissue cultures, particularly normal and tumoral kidney cell cultures, by means of conventional purification techniques. Human u-PA zymogen may play a similar physiological role, once activated to u-PA.

Recently it was reported that human u-PA was produced by DNA technology (see Eur. Pat. Appln. Publ. No. 92182).

The fibrinolytic activity of u-PA is determined by the fibrin plate method (see Plough J. et al. (1957) Biochim Biophys. Acta 24, 278) while the amidolytic activity on acetyl-lysyl-methyl-ester (ALME) is evaluated by a modification of the method of Sherry et al. (1964) J. Lab Clin Med 64, 145. Alternatively, the fibrinolytic activity of u-PA is determined by means of the synthetic substrate pyro-glutamyl-glycyl-argynyl-p-nitroanilide (S2444®).

0.1 ml sample, 0.1 ml porcine plasmin (Sigma, 25 microg/ml) and 0.8 ml of 0.1 M Tris.HCl buffer pH 8.8 containing 1% BSA are mixed followed by incubation for 1 h at 20°C. Samples are evaluated at different times for the amidolytic activity. The plasmin reaction is stopped by adding 100 microg of aprotinin (Richter) in 10 microl of buffer.

Both for the clinical assessment of the efficacy of pro-uPA and for the routine monitoring of plasma levels of treated patients, the importance of an accurate, precise, rapid and easy method like the method of the invention is evident to the man skilled in the art. Moreover, the present method is suitable for analyzing

EP 0 248 144 B1

u-PA and/or pro-uPA in complex media like cell and microorganism culture media.

Another possible application of the present method is in the monitoring, either in a biological fluid or in any aqueous medium, of the disappearance of high molecular weight u-PA (HMW), due for instance to conversion to low molecular weight u-PA (LMW) or any other degradation product thereof, by employing a monoclonal or polyclonal antibody preparation which is capable of recognizing HMW-uPA but not LMW-uPA, like monoclonal antibody 5B4 described below in more detail.

Other possible uses of the present invention will suggest themselves to the man skilled in the art reading the disclosure of the present invention and need not be discussed in great detail here.

The term "biological fluid" as used in the present application is therefore intended to encompass human biological fluids like plasma, urines, exudates, etc., as well as cell and microorganism culture media. Among these latter, culture media of DNA-recombinant microorganisms capable of producing u-PA and/or pro-uPA and culture media of u-PA and/or pro-uPA producing cells, like human epidermoid carcinoma cells , normal and tumoral human renal cells are particularly representative. It is also to be understood that all that is said about the assay in a "biological fluid" is intended and suitable for the assay in any solutions of the analytes.

For convenience, any solution of the analytes and any biological fluid which may be treated by the method of the invention will be referred to as "test solution" in the present application. With the term "analyte(s)", in the present description and claims, u-PA (human urinary plasminogen activator) and/or pro-uPA (its enzymatically inactive precursor) are intended.

More particularly, the method of the invention includes:

a) causing a test solution to contact with an analytical support bearing onto the surface a monoclonal antibody or a conventional antiserum which recognizes a common epitope on u-PA and pro-uPA without either inhibiting the activity of u-PA or inhibiting the enzymatic activation of pro-uPA

b) after rinsing, adding a buffered solution of an enzymatic activator of u-PA to a series of treated analytical supports while adding only the corresponding buffered solution to another series of treated analytical supports in parallel

c) determining the concentration of the analytes according to an amidolytic assay known per se on a synthetic color developing substrate by reference to a standard curve; the concentration of u-PA being directly determined from the samples which were not treated with the enzymatic activator in the foregoing step, while the concentration of pro-uPA being determined by difference between the concentration of total analyte measured in the samples treated with the enzymatic activator and the samples treated with the buffer only, in the foregoing step.

For the scope of the present application, the term "analytical support" refers to any analytical means which can contain or, at least, be exposed to liquid reagents. Preferred examples of these are glass or plastic microtiter plates or test tubes, plastic sheets, nitrocellulose and nylon membranes. The most preferred "analytical support" is represented by the wells of polyethylene, polystyrene, or polyvinylchloride microtiter plates.

Monoclonal antibodies against u-PA have been described since the pioneer work on monoclonal antibody-producing cell hybrids of C. Milstein and G. Köhler described in Nature, 256, 495-497 (1975).

For instance, monoclonal antibodies directed to urokinase (u-PA) are described in the following papers:
- Belgian Patent No. 896,253
- Bioscience Reports 3, 373-379 (1983)
- Thromb. Haemostas (Stuttgart) 49, 24-27 (1983)
- European Pat. Appln. Publication No. 0084344
- Proc. Natl. Acad. Sci. USA, 81, pages 110-114 (1984)
- Biochemistry, 21, 6410-6415 (1982)
- Proc. Natl. Acad. Sci. USA, 79, 3720-3723 (1982).

A monoclonal antibody which may be used in the method of the invention is a monoclonal antibody with the following characteristics:

a) it recognizes a common epitope on u-PA and pro-uPA and neither inhibits the activity of u-PA nor inhibits the enzymatic activation of pro-uPA

b) it has a good affinity for the antigen

c) it binds to u-PA and a single chain precursor thereof (such as pro-uPA)

d) it does not immunologically cross-react with serum or urine enzymatically active endopeptidases other than u-PA

For use in the method of the invention, the affinity of this monoclonal antibody for the antigen must be higher than $10^{-6}$, when measured essentially following the procedure described by Tsapis et al. in Eur. J. Biochem. 64, 369 (1976).

Preferably, this affinity, under the above conditions, is between $10^{-6}$ and $10^{-11}$, and most preferably

between $10^{-7}$ and $10^{-10}$.

A particularly preferred monoclonal antibody which may be used in the method of the invention is represented by a monoclonal antibody, named 5B4, which has the following characteristics:

a) it belongs to IgG$_1$ class

b) it has an affinity constant of about $1.42 \times 10^7$ l mole$^{-1}$, when measured essentially following the procedure described by Tsapis et al. in Eur. J. Biochem. 64, 369 (1976)

c) it specifically binds the high molecular form (54,000) of u-PA and a precursor thereof (such as pro-uPA) without binding the low molecular (33,000) form or any serum or urine thromboplastinic contaminant of u-PA

d) it binds the 18,000 Mr amino-terminal fragment of the A-chain

e) it does not impair the enzymatic activity of u-PA

f) it does not inhibit the enzymatic activation of pro-uPA

g) it has an isoelectric point of about 5.75

SDS-polyacrylamide-gel-electrophoresis (SDS-PAGE) of u-PA and pro-uPA in reducing and non reducing conditions was carried out according to Laemmli U.K. (1970) Nature 227, 680; immunoblotting experiments were carried out essentially as described previously (Salerno G. et al. (1984) Proc Natl Acad Sci USA 81, 110) using pure MAB 5B4, anti-uPA and anti-tissue PA antisera in the immune reaction.

The immunoblotting pattern of MAB 5B4 indicates interactions with HMW-uPA and with the proteolytic fragment (18,000) derived from the A-chain of HMW-uPA. This indicates that the epitope must be located on the A-chain. The fact that no interaction is observed by reduction of HMW-uPA with beta-mercaptoethanol indicates that the conformational changes induced by the reductive reagent destroy the epitope.

This monoclonal antibody, which has been disclosed in WO86/01411 is obtained by somatic cell fusion of mouse myeloma cells of the "non-secreting" type, i.e. myeloma cells which do not secrete immunoglobulins, with spleen cells of mice previously immunized with u-PA.

Briefly, Balb/c mice are immunized with highly purified 54,000 u-PA and the removed spleen cells are fused with non-secreting myeloma cells which possess certain genetic defects which make them uncapable of surviving in a certain medium which becomes very useful for the subsequent isolation of hybrid cells. An example of such cell line is that known as X63-Ag8653 which was described by Kearney et al., in J. Immunol. 123, 1548-1550 (1979) and which is known and available to the man skilled in the art.

Such myeloma cell lines are generally available to the public through many institutions of the scientific community such as The Salk Institute, Cell Distribution Center, P.O.Box 1809, San Diego, California 92112, and Institute for Medical Research, NIGMS Cell Repository, Copewood and Davis Streets, Camden, New Jersey 08103. The cell fusion is conducted in the presence of polyethylene glycol, preferably polyethylene glycol (PEG) 6000.

Highly purified 54,000 u-PA (HMW u-PA) is commercially available from many sources. A suitable highly purified u-PA preparation has an high titer in International Units (IU), a high ratio fibrinolytic/esterolytic activity and very low amounts or a practical absence of thromboplastinic contaminants. A preferred highly purified u-PA preparation is that commercialized under the trade name PERSOLV® (Gruppo Lepetit S.p.A.) which has a fibrinolytic activity higher than 100,000 IU/mg, a ratio fibrinolytic/esterolytic activity higher than 1.4, is apyrogen in rabbits at doses higher than 20,000 IU/Kg and has a zero-coagulant activity at plasma concentrations as high as 200 IU/ml.

The immunization schedule is preferably characterized by a low dose administration of u-PA. A typical immunization schedule in mice includes a first administration of a low dose of highly purified 54,000 MW u-PA (120,000 IU/mg; about 10 μg) in complete Freund adjuvant 60 days before fusion, i.p.; a second i.p. injection of the same amount in incomplete Freund adjuvant 30 days before fusion and a final i.v. booster of the same amount 5 days before fusion.

Only those animals which 10 days before fusion have an anti-uPA antibody titer higher than 1:10,000 are given the final booster and further processed.

Spleens are removed and the spleen cells are fused with the myeloma cells at a ratio of about $10^8$ to $5 \times 10^7$ cells. After fusion, the cells are cultured in a suitable medium such as Dulbecco's modified Eagle medium containing about 10% fetal calf serum and the fused cells are selected on a Hypoxanthine, Aminopterine, Thymidine medium (HAT). The supernatants of the cell cultures are assayed for anti-uPA antibodies by means of ELISA methods involving peroxidase labelled anti-mouse immunoglobulin "conventional" antiserum. The purification of the thus identified anti-uPA monoclonal antibodies can be conducted by means of ion-exchange chromatography or by affinity chromatography on a urokinase-agarose column, while gel electrophoresis is a convenient mean for evaluating the homogeneity of the obtained product. These preparations of anti-uPA monoclonal antibodies are then evaluated for class specificity and affinity constant for the antigen immobilized on agarose. The class specificity is evaluated according to the Ouchterlony's double immunodiffusion technique (Acta Pathol. Microbiol. Scand. 26, 507, (1949)); the affinity constant is determined by equilibrium binding experiments conducted essentially following the method of Tsapis et al. (Tsapis A., Rogard N., Alfsen A., and Nihaesco C. (1976) Eur. J. Biochem. 64, 369). In these experiments increasing concentrations of essentially pure monoclonal antibody previously dialyzed in phospha te buffered saline pH 7.2 are added to an urokinase-bearing matrix, such as a uPA-agarose matrix. The concentration of the unbound antibody is determined spectrophotometrically, the concentration of bound antibody is calculated by difference from the total antibody added, and the binding data are elaborated according to the known statistical methods such as the method described by G. Scatchard in Ann. N.Y. Acad. Sci. 51, 660-672 (1949).

The coating of the analytical support with the monoclonal antibody or conventional antiserum is obtained by contacting a solution of the antibody dilution or the antisera wherein the amount of antibody is largely in excess of the amount of antigen-analyte to be detected. Possibly uncoated surface is saturated by adding an excess of a non-interfering protein solution such as bovine serum albumine.

After coating, the analytical support is shaken dry and stored at about 4°C until use.

A preferred analytical support is a polyvinyl chloride microplate, such as Falcon Microtest III® flexible assay plate of Becton-Dickinson, USA, wherein each well has been contacted with from 0.5 $\mu$g/ml to 50 $\mu$g/ml of a solution of monoclonal antibody 5B4.

The "enzymatic activator" is one of the known substances which are capable of transforming pro-uPA (a single chain enzymatically inactive zymogen) into u-PA (the two-chain active enzyme).

Examples of said enzymatic activators of pro-uPA are trypsin-like endopeptides such as plasmin, trypsin, thrombin and kallicrein.

Among them, plasmin is the preferred activator which selectively splits the peptidic chain in the region around a lysine (Lys) group at position 158 (see S. Kasai et al., J. Biol. Chem., 22, 12383-12389 (1985)).

This enzymatic activation transforms pro-uPA in an activated form which has an enzymatic activity very similar to u-PA and at least in the case of plasmin activation, there is strong evidence that the enzymatically active product is in fact u-PA.

"Amidolytic assays" for u-PA are known in the art. For the method of the invention, those amidolytic assays which involve the hydrolysis of a chromogenic substrate are preferred.

Many different substrates of u-PA of this type are known in the art and commercially available. In the present method one of the preferred is the synthetic oligopeptide derivative, pyro-glutamyl-glycyl-arginyl-p-nitroanilide (pyro-Glu-Gly-Arg-pNA) commercialized by Kabi-Sweden with the acronym S2444.

"Standard" preparations of u-PA and/or pro-uPA are known and can also be obtained by a purification method which involves the use of monoclonal antibody 5B4 immobilized on an affinity chromatography support.

Validation experiments indicate that intra- assay precision (CV%) of the method of the invention varies from 3% to 7%, while the inter- assay precision (CV%) is generally from 5% to 10%. Analytical recovery ranges from 90% to 110%.

The application range is from 1 to 100 IU/ml of u-PA and preferably from 1.5 IU/ml to 55 IU/ml.

Pro-uPA concentration is expressed as the corresponding equivalent u-PA international units (IU)/ml evaluated after enzymatic activation.

The following examples and preparations further illustrate the invention.


Example 1:


Immunoadsorbent amidolytic assay


A431 cell supernatants which contain both uPA and pro-uPA are diluted 2-fold with a solution of 0.3 M sodium chloride and 0.1 M sodium phosphate buffer pH 7.3 containing 60 g/l of bovine serum albumine (BSA) and 0.1% Tween® 20 (polyoxyethylene sorbitan monolaurate). The reference standard (HMW-uPA) is diluted at various concentrations (52.5, 26.25, 13.12, 6.562, 3.281 IU/ml) in 0.15 M sodium chloride, O.05 M sodium phosphate buffer pH 7.3 (PBS) containing 0.05% Tween® 20, 30 g/l BSA and 50% Dulbecco's Modified Eagle Medium DMEM (Flow Laboratories, USA). Aliquots (200 $\mu\ell$) of each standard and samples are added in triplicate to different wells of a 5B4-coated microplate prepared as described in preparation 8. The solutions are let to contact for 2 h at room temperature in a humid covered box. Then the plate is washed eight times by empting and filling with PBS containing 0.05% Tween® 20 (PBS-T buffer) and shaken dry. To evaluate the total amidolytic activity (u-PA plus pro-uPA) 200 $\mu\ell$/well of 10 $\mu$g/ml plasmin solution in 38 mM sodium chloride, 50 mM TRIS buffer pH 8.8 containing 10 g/l BSA ("S2444 buffer") is added to the wells. Only "S2444 buffer" is added to the wells where u-PA should be evaluated. After

5

incubating for 1h at room temperature the plasmin catalyzed reaction is stopped by adding 25 $\mu\ell$/well of a 0.35 mg/l aprotinin solution in distilled water. The solution in each well is thoroughly mixed by means of a multichannel pipette and left to stand for 5 min at room temperature. Then 25 $\mu\ell$/well of a solution of u-PA synthetic substrate S2444 (Kabi, Sweden) (1.5 mg/ml in distilled water) is added and mixed thoroughly as indicated above. The colour is let to develop for two hours at 37°C and read at 405 nm in a Titertek Multiskan Photometer (Flow Laboratories, USA). u-PA concentration and the total concentration of u-PA plus pro-uPA in the samples are obtained by interpolating the optical densities of the wells untreated and treated with plasmin, respectively, on the dose-response curve of standards. The concentration of pro-uPA is calculated by difference between the "total" concentration and the u-PA concentration so obtained.

The results of a representative assay are reported in Tables I, II, and III.

More particularly, table I reports the intra- and inter-assay precision of measurements of u-PA and pro-uPA by the assay exemplified above; table II reports the analytical recovery of an equimolecular mixture of u-PA and pro-uPA added to the A431 cell supernatants as obtained by the assay exemplified above, and table III reports the concentration of u-PA and pro-uPA as measured according to the above assay at different dilutions of the test sample.

## TABLE I

### INTRA- AND INTER-ASSAY PRECISION IN A431 CELL SUPERNATANTS (n=8)

| | u-PA, IU/ml | | | pro-uPA, IU/ml | | |
|---|---|---|---|---|---|---|
| | Mean | SD | CV% | Mean | SD | CV% |
| **Intra-assay** | | | | | | |
| A | 1.59 | 0.10 | 6.780 | 13.6 | 0.93 | 6.860 |
| B | 7.7 | 0.33 | 4.284 | 20.7 | 1.518 | 7.329 |
| Mean | | | 5.530 | | | 7.090 |
| **Inter-assay** | | | | | | |
| A | 1.69 | 0.26 | 15.540 | 13.1 | 0.945 | 7.190 |
| B | 8.25 | 0.62 | 7.590 | 21.0 | 1.819 | 8.647 |
| C | 5.43 | 0.308 | 5.663 | 25.0 | 1.987 | 7.940 |
| D | 1.61 | 0.174 | 10.837 | 29.37 | 1.603 | 5.461 |
| E | ud | – | – | 26.85 | 2.419 | 8.970 |
| F | ud | – | – | 27.1 | 2.91 | 10.700 |
| G | ud | – | – | 3.84 | 0.384 | 9.990 |

TABLE I (continued)

INTRA- AND INTER-ASSAY PRECISION IN A431 CELL SUPERNATANTS

| | u-PA, IU/ml | | | pro-uPA, IU/ml | | |
|---|---|---|---|---|---|---|
| | Mean | SD | CV% | Mean | SD | CV% |
| Inter-assay | | | | | | |
| H | ud | - | - | 3.3 | 0.28 | 8.760 |
| I | ud | - | - | 4.40 | 0.48 | 10.908 |
| L | 3.2 | 0.178 | 5.557 | 8.66 | 0.582 | 6.722 |
| Mean | | | 9.037 | | | 8.529 |

n  = 8 each

ud  = undetectable

SD  = standard deviation

CV% = coefficient of variation

EP 0 248 144 B1

## TABLE IIa

### ANALYTICAL RECOVERY OF AN EQUIMOLAR

### MIXTURE OF u-PA AND PRO-uPA ADDED TO A431 CELL SUPERNATANTS

| Added [b] | Amidolytic activity [a], IU/ml | | O/E [d], % |
|---|---|---|---|
| | Expected[c] | Observed | |
| 300 | 398.1 | 459.6 | 115.4 |
| 150 | 248.1 | 290.2 | 104.5 |
| 75 | 173.1 | 175.5 | 101.4 |
| 37.5 | 135.6 | 107.5 | 107.5 |
| 18.75 | 116.85 | 115.8 | 99 |

Total Activity

Mean 105.5

a) Evaluated by S2444 hydrolysis after plasmin treatment

b) As an equimolar mixture of u-PA and pro-uPA

c) Initial concentration in A431 cell supernatant, 98.1IU/ml

d) O/E = observed/expected

EP 0 248 144 B1

## TABLE IIb

### ANALYTICAL RECOVERY OF u-PA AND PRO-uPA

### ADDED TO A431 CELL SUPERNATANTS

| Sample A u-PA added (IU/ml) | Recovery (%) | Sample B pro-uPA added (IU/ml) | Recovery (%) |
|---|---|---|---|
| 46.5 | 100.5 | 18.8 | 103.0 |
| 23.3 | 99.6 | 9.3 | 89.1 |
| 11.6 | 92.9 | 4.6 | 93.7 |
| 5.8 | 89.9 | 2.3 | 102.2 |
| 2.9 | 93.0 | – | – |
| | Mean 95.2 | | Mean 96.9 |

u-PA initial concentration was undetectable both in sample A and B;
pro-uPA initial concentration was 42.56 IU/ml in sample A and 5.32 IU/ml
in sample B

## TABLE III

### u-PA AND PRO-uPA CONCENTRATIONS
### IN A431 CELL SUPERNATANTS AT VARIOUS DILUTIONS

| Dilution ratio | u-PA, IU/ml | | | pro-uPA (IU/ml) | | |
|---|---|---|---|---|---|---|
| | Expected | Observed | O/E% | Expected | Observed | O/E% |
| 1.5 | 31.89 | 30.57 | 95.2 | 53.36 | 56.03 | 105 |
| 2.25 | 31.89 | 31.89 | 100.0 | 53.36 | 56.19 | 105.3 |
| 3.375 | 31.89 | 34.1 | 107.1 | 53.36 | 49.03 | 91.9 |
| 5.0625 | 31.89 | 33.78 | 105.9 | 53.36 | 58.1 | 108.8 |

By repeating the above experiments using urine or a fermentation broth containing u-PA and/or pro-uPA substantially the same results of precision, accuracy and sensitivity are obtained.

In particular, validation experiments indicate that intra- assay precision (CV%) varies from 3% to 7%; while inter-assay precision (CV%) is generally from 5% to 10%. Analytical recovery ranges from 90% to 110%. The application range is from 15 mg/ml to 100 mg/ml of analyte.

Preparation 1:

Preparation of anti-uPA monoclonal antibody 5B4

a) Production of anti-uPA monoclonal antibodies

Highly purified 54,000 Dalton u-PA 120,000 IU/mg (PERSOLV ® RICHTER) is used to immunize 7-week old female Balb/C mice. 10 $\mu$g of this u-PA in complete Freund's adjuvant is injected into the peritoneum of the animals 60 days before fusion. Another 10 $\mu$g of u-PA is given to mice 30 days later. Ten days before fusion, anti-uPA antibody titer is measured by conventional ELISA method in blood samples taken from the tail veins and 5 days before fusion, a final booster of 10 $\mu$g of u-PA is given i.v. only to those animals having an antibody titer higher than 1:10,000. Spleens are then removed on the day of the fusion and spleen cells (about $1 \times 10^8$) are fused to $5 \times 10^7$ mouse myeloma X63-Ag8653 in 50% PEG 6000.

After fusion, which has been performed substan-tially following the method of C. Milstein and G. Köhler, the cells are resuspended in Dulbecco's Modified Eagle Medium (DMEM) supplemented with 10% fetal calf serum and HAT medium (0.1mM Hypoxanthine, 0.25 mM Aminopterine, 0,017 mM Thymidine, Flow laboratories) and put onto 5 different Costar plates containing mouse macrophages (feeder layer).

The HAT medium is changed every 3 days and from day 10 after fusion the supernatants are tested in an ELISA immunoassay every 2-3 days for the presence of anti-uPA monoclonal antibodies.

b) Screening of anti-uPA monoclonal antibody-producing hybridomas by ELISA

According to a modification of the Perlman method (Immunochemistry, 8, 873 (1971)), flexible 96 well microtiter plates (Dynatech) are coated with 50 $\mu\ell$/well of a 20 $\mu$g/ml solution of u-PA in phosphate buffered saline pH 7.2, 0.05 M sodium phosphate pH 7 which contains 0.5 M sodium chloride and incubated for 1 h at room temperature. After a thorough washing with 0.05% Tween® 20 phosphate buffered saline, the wells are saturated with 3% bovine serum albumine phosphate buffered saline, for 3 h at room temperature to avoid unspecific binding. After washing carefully, 50 $\mu\ell$ of supernatant hybridoma cultures (or the same volume of ascites fluids) are added to the wells and left to react two hours at room temperature. The plates are then washed and incubated with a 1:1000 dilution of rabbit-anti-mouse Ig conjugated with horseradish peroxidase (PI61, DAKO), for 90 min at room temperature. After thorough washing, the plates are incubated with 200 $\mu\ell$/well of 1 mg/ml solution of the chromogen o-phenylendiamine (SIGMA) in O.1M citrate buffer pH 5, 1.7 mM $H_2O_2$. The color is developed in 20 minutes and the optical density read at 492 nm against blanks with neither antigen nor antibody. Wells with color levels 4 times higher than the blanks are considered positive.

c) Mass cultivation of anti-uPA producing hybridomas

Anti-uPA antibody producing hybridomas are selected, cloned by limiting dilution, grown in mass cultures and injected into the peritoneum of Balb/c mice previously treated with 0.5 ml of pristane ® (2,6,10,14-tetramethylpentadecane; Aldrich). Ascite fluids are collected 15 days later. The concentration of anti-uPA monoclonal antibodies is in the range of 10-20 mg/ml. They are purified either by protein-A Sepharose or urokinase-Sepharose affinity chromatography.

d) Purification of anti-uPA monoclonal antibodies by affinity chromatography on Sepharose ® -uPA

A Sepharose-uPA conjugate is prepared by reacting CNBr-activated Sepharose HMW with highly purified u-PA (120,000 IU/mg). The coupling efficiency is of about 20 mg u-PA/ml swollen gel. Ascite fluids obtained as above (1 ml per producing hybridoma) are then applied to the Sepharose-urokinase affinity column (1.6 cm $\times$ 15 cm) pre-equilibrated in 0.01 M sodium phosphate buffer pH 8.0. After rinsing with the same buffer, the selectively adsorbed anti-urokinase monoclonal antibodies are eluted with 0.1 M acetic acid pH 3.0. The antibody containing fractions are pooled and concentrated by ultrafiltration on PSDE 09005® Millipore membranes and stored frozen until use.

e) Evaluation of the purity and homogeneity of the monoclonal antibody preparations by gel-electrophoresis

The monoclonal antibody obtained above is submitted to SDS-PAGE (sodium dodecylphosphate polyacrylamide gel eletrophoresis) according to the method described by U.K. Laemmli in Nature 227, 680 (1970). Only the bands corresponding to the heavy and the light chains of the IgG are evident indicating that the above eluate contains pure immunoglobulins.

f) Determination of the affinity constant

Aliquots of a 20% Sepharose-uPA suspension in phosphate buffered saline pH 7 (0.5 ml each) are added to increasing concentrations of purified anti-uPA monoclonal antibody preparations (these preparations are those obtained according to point d, above, dialyzed in phosphate buffered saline pH 7). The samples are rotated end over end for two hours at room temperature. The controls received no resins.

The concentration of the unbound antibody is spectrophotometrically determined at 280 nm

$$(E_{1cm}^{1\%} = 14),$$

while the concentration of the bound antibody is calculated by difference from the total amount of added antibody. All binding data are elaborated according to Scatchard (Ann. N.Y. Acad. Sci., 51, 660-672, 1949). The monoclonal antibody denominated 5B4 has an affinity constant of $1.42 \times 10^7$ l mole$^{-1}$.

Preparation 2

Purification of anti-uPA immunoglobuline (IgGs) from an anti-UK conventional antiserum

Rabbit anti-UK antiserum was obtained according to usual immunization procedures by using highly purified 54,000 u-PA (about 120,000 IU/mg).

More particularly, healthy New Zealand white rabbits are immunized with 500 $\mu$g of highly purified 54,000 u-PA (about 120,000 IU/mg) in complete Freund's adjuvant. Booster injections of the same amount of antigen in incomplete Freund's adjuvant are administered after 4, 6 and 8 weeks. Animals are bled weekly and anti-uPA antibody titers are determined using anti-uPA antiserum enzyme - Immunoassay. After 12 weeks animals are bled completely and IgG immunoglobulin fraction is purified by affinity chromatography on protein-A Sepharose. One ml of the obtained antiserum is applied to a Protein A - Sepharose column (column size: 1.6 cm $\times$ 4 cm) previously equilibrated with 0.1 M sodium phosphate buffer pH 8.0 at a flow rate of 20 ml/h. After washing with the same buffer, IgGs are eluted with 0.1 M acetic acid pH 3. Fractions of 2 ml are collected and IgGs presence is monitored by enzyme immunoassay. Fractions containing IgGs are adjusted to pH 7.2 and stored frozen at -20°C until use.

Preparation 3:

Preparation of A431 cell supernatant

Human epidermoid carcinoma A431 cells are cultivated essentially as described by Fabrian et al. Proc. Natl. Acad. Sci., USA, 74, 565 (1977) . More particularly, A431 cells are grown to confluency in Dulbecco's modified Eagle medium containing 10% fetal calf serum (Flow Laboratories), 100 units/$\mu$l of penicillin, 100 $\mu$g/ml of streptomycin (Flow Labs.) and 2 mM of glutamine (Flow Labs.) in plastic culture flasks 150 cm$^2$ growth surface (NUNC). Then the supernatants are collected, centrifuged to eliminate the debris and stored at -80°C in the presence of a protease inhibitor (aprotinin, 10 $\mu$g/ml) until use.

Preparation 4:

Immobilization of anti-uPA monoclonal antibody 5B4 on activated agarose

Activated agarose (N-hydroxysuccinimide active ester derivative of cross-linked agarose beads having a 10 atom long spacer arm; Affi-gel 10 ®, Bio-rad Inc.; 14 g, wet) is washed with 3 volumes of isopropylic alcohol and 3 volumes of cold distilled water in 20 minutes. The gel is then resuspended in 0.1 M sodium bicarbonate buffer pH 8 (final volume = 20 ml). Highly purified monoclonal antibody 5B4, as obtained above, in 0.1 M sodium bicarbonate buffer pH 8 (9.7 ml) is added to the gel suspension. After four hours at room temperature 1M aqueous ethanolamine hydrochloride at pH 8 (1,5 ml) is added to block the unreacted ester groups of the resin. The blocking reaction is completed in one hour at room temperature. The gel is

13

then packed into a column, washed with 10 volumes of the coupling buffer (0.1 M aqueous sodium bicarbonate pH 8), then with 0.1 M aqueous acetic acid followed by 0.1 M aqueous acetic acid containing 1 M aqueous sodium chloride and lastly with 0.05 M aqueous sodium phosphate buffer pH 7 containing 0.5 M aqueous sodium chloride. The efficiency of the coupling is evaluated spectrophotometrically at 280 nm in the supernatant samples before and after reaction. These samples are brought to pH 2 before assaying to eliminate the interference of N-hydroxy succinimide in the readings.

2.5-4 mg of monoclonal antibody 5B4 were immobilized per ml of resin.

Preparation 5:

a) Evaluation of the capacity of the 5B4-Agarose adsorbent

The maximum capacity of 5B4-Agarose is evalutated on "pure" and crude preparations of u-PA. A column of 5B4 Agarose (1.6 × 10 cm) is equilibrated in 0.5 M NaCl, 0.05 M Na-phosphate buffer pH 7 and loaded with "pure" u-PA (120,000 IU/mg) until esterolytic activity is detected in the effluent. The column is then washed with the equilibrating buffer and the enzyme is desorbed by eluting with 1 M NaCl, 0.1 M acetic acid. The total activity recovered in the bound fraction is taken as the maximum capacity of the column.

In the case of u-PA (120,000 IU/mg) the capacity of 5B4 agarose was 216,000 IU/ml.

The same experiment was repeated using a crude preparation of u-PA (700 IU/mg) and a capacity of 174,000 IU/ml was obtained.

b) Immunoadsorption of pro-uPA HMW-uPA, and 18,000 u-PA Mr amino-terminal fragment on 5B4 agarose.

Purified HMW u-PA (30 mg) is incubated for 8 h in 1 ml of 50 mM sodium phosphate buffer pH 8 containing 0.2 M NaCl.HMW-uPA, LMW-uPA and 18,000 proteolytic fragment are obtained in a ratio of about 4:4:2, respectively. These products are separated and isolated by gel-filtration on a cross-linked controlled pore polydextran (Sephadex ® G 100, superfine; 1.5 × 100 cm) equilibrated in 5 mM sodium phosphate buffer pH 4 containing 0.2 M NaCl and eluted with the same mixture at a flow rate of 3 ml/h.

The eluted fractions containing the single products are then collected. Suspensions of scalar amounts of 5B4-agarose (prepared as described above) in phosphate buffered saline pH 7.5 containing 0.1% (v/v) Tween ® 20 are added to test tubes containing 0.5 ml of pro-uPA HMW-uPA, LMW-uPA or 18,000 Mr amino-terminal fragment u-PA solutions (250 microg/ml each) in the same buffer and the final volume is brought to 1.5 ml with the same buffer.

All samples are rotated end over end for 1 h at room temperature and then centrifuged for 5 min at 2000 rpm. The unbound material is determined by reading the optical density of the supernatants at 280 nm; the bound material is determined by difference with the total added.

In these experiments, more than 90% of the pro-uPA and HMW-uPA and 18,000 Mr amino-terminal fragment are immunoadsorbed on 5B4-agarose whereas LMW u-PA is not immunoadsorbed.

Preparation 6:

Purification of u-PA by immunoaffinity on 5B4-Agarose

A chromatography column (1.6 × 9 cm) is filled with the 5B4-Agarose immunoadsorbent as obtained according to preparation 4 and equilibrated with 0.5 M aqueous sodium chloride and 0.05 M aqueous phosphate buffer pH 7. A crude urinary concentrate is then loaded at a flow rate of 60 ml/h. After washing with the equilibrating buffer, u-PA is eluted with 1 M aqueous sodium chloride and 0.1 M aqueous acetic acid.

The u-PA containing fractions are pooled, neutralized and freeze-dried.

The specific activity of the recovered u-PA was always greater than 130,000 IU/mg.

The quantitative data are reported in the following table:

TABLE IV

| FRACTION | VOL. | TOTAL FIBRINOLYTIC ACTIVITY [a] | % | TOTAL ESTEROLYTIC ACTIVITY [b] | TOTAL PROTEIN | SPEC. ACTIVITY | PURIF. FACTOR | IU/EU |
|---|---|---|---|---|---|---|---|---|
| | ml | IU | | EU | mg | IU/mg | | |
| CRUDE EXTRACT | 186 | 1772394 | 100 | 897 | 2007 | 883 | – | – |
| UNBOUND FRACTION (A) | 270 | 141791 | 8 | 164 | – | – | – | 846 |
| BOUND FRACTION (B) | 56 | 1374240 | 77.5 | 683 | 9.52 | 144353 | 163 | 2012 |

a) evaluated essentially following the method of the 1979 Addendum of the British Pharmacopoea

b) evaluated essentially following the method described Sherry et al., J. Lab. Clin. Med., $\underline{64}$, 145 (1964) on AlME

EP 0 248 144 B1

TABLE V

| | |
|---|---|
| Thromboplastinic contaminants: | zero-coagulation at 193 IU/ml plasma (zero-coagulation value of the National Heart and Lung Institute, U.S.A.: 80 IU/ml plasma) |
| Acute toxicity in mouse: | non toxic at 100,000 IU/kg, i.v. |
| Systemic tolerability in dog: | at 20,000 IU/kg there was no significant change in arterial blood pressure |

The HPLC analysis revealed the presence of high molecular weight (54,000) u-PA and the absence of low molecular weight (33,000) u-PA.

The purity of the obtained u-PA was also confirmed by gel electrophoresis on sodium dodecylsulfate polyacrylamide. Also this test, in fact, confirms that the u-PA product purified on 5B4-agarose is essentially 54,000 MW u-PA.

The experiments were repeated several times (more than 50 cycles) without any significant change in the results.

Consistently, 70-80% of the fibrinolytic activity loaded onto the column is detected in the bound fractions. When the unbound fractions are reloaded onto the column all the activity is detected in the flow-through suggesting that this activity may not be due to u-PA but rather to unspecific proteases with fibrinolytic activity unrelated to u-PA.

Preparation 7

Purification of pro-uPA

A 5B4 column (1.6 × 10 cm) prepared as described in preparation 1 is equilibrated with 0.15 M NaCl, 0.5 M sodium phosphate buffer (pH 7) containing 0.1% Triton X-100® (Rohm and Haas). The column is loaded with 10 l of A431 cell supernatants (see preparation 3) at a flow rate of 200 ml/h, at 4°C and then washed with the equilibrating buffer. Desorption is carried out by eluting with 1 M NaCl, 0.1 M acetic acid containing 0.1% Triton X-100®. Fractions are collected and tested by the amidolytic assay on S2444 substrate before and after activation with plasmin as described above. Fractions containing pro-uPA are collected. The pooled solution is further purified and concentrated by ion-exchange chromatography on FPLC (Pharmacia, Sweden) using a Mono S HR5/5 cation exchange column (5 × 50 mm) equilibrated with 50 mM sodium acetate buffer (buffer A), pH 4.0. The sample is two-fold diluted with distilled water and loaded onto the column. After washing with the equilibrating buffer the bound pro-uPA is eluted with a salt/pH gradient (from 0.3 M NaCl in buffer A at pH 4.0 to 0.7 M NaCl in buffer A at pH 5.0, linear gradient in 50 min, flow-rate : 60 ml/h) and finally with buffer A containing 1 M NaCl, pH 5.0. The eluted fractions are monitored by means of the above described amidolytic assay on S2444 and the pro-uPA containing fractions are pooled and kept frozen.

The obtained product (pro-uPA) appears to be homogeneous in SDS-PAGE.

SDS-PAGE, Western blotting analysis and zymography show that pro-uPA purified as above is a single chain protein of about 48.000 immunologically related to the urinary-type PA and distinct from the tissue-type PA. The active site appears to be masked in pro-uPA, since no incorporation of [3H] DFP (3H-diisopropylfluorophosphate) and no binding to benzamidine-agarose is observed.

Moreover, this pro-uPA is inactive on synthetic substrate S2444 but plasmin treatment converts it into a two-chain active form (150,000 IU/mg) similar to u-PA. This activated form incorporates 3H-DFP in the 31,500 heavy chain and binds to benzamidine-agarose. The amino-terminal sequence (26 aminoacids) of pro-uPA purified as above from A431 cells proved to be identical to that of pro-uPA purified from kidney cells (J. Biol. Chem. 22, 12383-12389 (1985)).

Preparation 8:

Coating of microtiter plates with 5B4

Each well of a polyvinyl chloride microtiter plate (Falcon MicroTest III flexible assay plate; Becton Dickinson and Co., USA) is filled with 200 microl of a 35 microg/ml 5B4 solution in 0.15 M sodium chloride, 0.05 M sodium phosphate buffer, pH 7.3 (PBS). After incubating for 2 h at room temperature in a covered box, the microtiter plates are washed five times by emptying and filling with PBS. Then 250 microl of a 10

EP 0 248 144 B1

g/l bovine serum albumine (BSA) solution in PBS was added to each well and let this stand for 2 h at room temperature to block the uncoated plastic surface. The plates are washed again with PBS, shaken dry and stored at 4°C until use.

By following substantially the same procedure but using a conventional antiserum such as that obtained according to preparation 2 an antiserum coated microtiter plate is obtained which can be used in the method of example 1.

**Claims**

1. A method for determining an analyte selected from human urinary plasminogen activator (u-PA) and a zymogen thereof (human urinary plasminogen activator precursor, pro-uPA), or a mixture thereof which comprises:

   a) causing a test solution to contact with an analytical support bearing onto the surface a monoclonal antibody or a conventional antiserum which recognizes a common epitope on u-PA and pro-uPA without either inhibiting the activity of u-PA or inhibiting the enzymatic activation of pro-uPA

   b) after rinsing, adding a buffered solution of an enzymatic activator of u-PA to a series of treated analytical supports while adding only the corresponding buffered solution to another series of treated analytical supports in parallel

   c) determining the concentration of the analytes according to an amidolytic assay known per se on a synthetic color developing substrate by reference to a standard curve; the concentration of u-PA being directly determined from the samples which were not treated with the enzymatic activator in the foregoing step, while the concentration of pro-uPA being determined by difference between the concentration of total analytes measured in the samples treated with the enzymatic activator and the samples treated with the buffer only, in the foregoing step.

2. A method according to claim 1 wherein the analytical support is made of plastic.

3. A method according to claim 1 wherein the analytical support is selected from plastic microtiter plates, test tubes or sheets or nitrocellulose or nylon membranes.

4. A method according to claim 1 wherein the analytical support is a polystyrene, polyvinylchloride or polyethylene microtiter plate.

5. A method according to claim 1 wherein the analytical support has been coated with a monoclonal antibody or conventional antiserum having the following characteristics:

   a) it recognizes a common epitope on u-PA and pro-uPA and neither inhibits the activity of u-PA nor inhibits the enzymatic activation of pro-uPA

   b) it has an affinity for the antigen between $10^{-6}$ and $10^{-11}$.

   c) it binds to u-PA and a single chain precursor thereof (such as pro-uPA)

   d) it does not immunologically cross-react with serum or urine enzymatically active endopeptidases other than u-PA.

6. A method according to claim 1 wherein the analytical support has been coated with a monoclonal antibody having the following characteristics:

   a) it belongs to IgG$_1$ class

   b) it has an affinity constant of about 1.42 x $10^7$ l mole$^{-1}$ (antigen urokinase-bearing matrix), when measured essentially following the procedure described by Tsapis et al. in Eur. J. Biochem. 64, 369 (1976)

   c) it specifically binds the high molecular form (54,000) of u-PA and a precursor thereof (such as pro-uPA) without binding the low molecular (33,000) form or any serum or urine thromboplastinic contaminant of u-PA

   d) it binds the 18,000 Mr amino-terminal fragment of the A-chain

   e) it does not impair the enzymatic activity of u-PA

   f) it does not inhibit the enzymatic activation of pro-uPA

   g) it has an isoelectric point of about 5.75

7. A method according to claim 1 wherein the enzymatic activator is selected from plasmin, thrombin, kallicrein and trypsin.

17

**8.** A method according to claim 1 wherein the amidolytic reaction employs a color developing substrate for u-PA.

**9.** A method according to claim 1 wherein the amidolytic reaction employs a color developing substrate for u-PA which is pyro-glutamyl-glycyl-Arginyl-p-nitroanilide.

**10.** A kit for use in the method of claim 1 which includes:
a) an analytical support bearing onto the surface a monoclonal antibody or a conventional antiserum which recognizes a common epitope on u-PA and pro-uPA and neither inhibit the activity of u-PA nor inhibit the enzymatic activation of pro-uPA
b) an enzymatic activator of pro-uPA, and optionally
c) a chromogenic substrate for u-PA.

**Patentansprüche**

**1.** Verfahren zur Bestimmung eines Analyten, ausgewählt aus Human-Urin-Plasminogen-Aktivator (u-Pa) und einem Zymogen davon (Human-Urin-Plasminogen-Aktivator-Vorläufer, pro-uPA), oder einem Gemisch davon, dadurch **gekennzeichnet, daß** man:
a) eine Testlösung in Kontakt mit einem analytischen Träger, welcher auf der Oberfläche einen monoklonalen Antikörper oder ein übliches Antiserum trägt, welches ein gemeinsames Epitop auf u-PA und pro-uPA erkennt ohne entweder die Aktivität von u-PA zu inhibieren oder die enzymatische Aktivierung von pro-uPA zu inhibieren, bringt
b) nach Spülen eine gepufferte Lösung eines enzymatischen Aktivators von u-PA einer Reihe von behandelten analytischen Trägern zusetzt, während man nur die entsprechende gepufferte Lösung einer weiteren Serie von behandelten analytischen Trägern parallel dazu zusetzt
c) die Konzentration des Analyten gemäß einem an sich bekannten amidolytischen Assay auf ein synthetisches farbentwickelndes Substrat unter Bezug auf eine Standardkurve bestimmt, wobei die Konzentration von u-PA direkt von den Proben, welche mit dem enzymatischen Aktivator in dem vorausgehenden Schritt nicht behandelt wurden, bestimmt wird, während die Konzentration von pro-uPA durch Differenz zwischen der Konzentration der Gesamtanalyten, die in den mit dem enzymatischen Aktivator behandelten Proben gemessen wurde und den Proben, die nur mit Puffer in dem vorausgehenden Schritt behandelt wurden, bestimmt wird.

**2.** Verfahren nach Anspruch 1, dadurch **gekennzeichnet,** daß der analytische Träger aus Plastik besteht.

**3.** Verfahren nach Anspruch 1, dadurch **gekennzeichnet,** daß der analytische Träger ausgewählt wird aus Plastik-Mikrotiter-Platten, Reagensgläsern oder Platten aus Nitrocellulose oder Nylonmembranen.

**4.** Verfahren nach Anspruch 1, dadurch **gekennzeichnet,** daß der analytische Träger eine Polystyrol-, Polyvinylchlorid- oder Polyethylen-Miktrotiter-Platte ist.

**5.** Verfahren nach Anspruch 1, dadurch **gekennzeichnet,** daß der analytische Träger mit einem monoklonalen Antikörper oder einem üblichen Antiserum mit den folgenden Eigenschaften beschichtet worden ist:
a) er bzw. es erkennt ein gemeinsames Epitop auf u-PA und pro-uPA und hemmt weder die Aktivität von u-PA noch die enzymatische Aktivierung von pro-uPA
b) er bzw. es besitzt eine Affinität für das Antigen zwischen $10^{-6}$ und $10^{-11}$
c) er bzw. es bindet an ein u-PA und an den einkettigen Vorläufer davon (beispielsweise pro-uPA)
d) er bzw. es zeigt keine immunologische Kreuzreaktion mit enzymatisch aktiven Serum- oder Urin-Endopeptidasen außer u-PA.

**6.** Verfahren nach Anspruch 1, dadurch **gekennzeichnet,** daß der analytische Träger mit einem monoklonalen Antikörper mit den folgenden Eigenschaften beschichtet worden ist:
a) er gehört zu der Klasse $IgG_1$
b) er besitzt eine Affinitätskonstante von etwa $1,42 \times 10^7$ l $mole^{-1}$ (Antigen-Urokinase-tragende Matrix), bei Messung im wesentlichen nach dem von Tsapis et al. in Eur. J. Biochem. 64, 369 (1976) beschriebenen Verfahren
c) er bindet spezifisch an die hochmolekulare Form (54000) von u-PA und an einen Vorläufer davon (beispielsweise pro-uPA) ohne an die niedermolekulare Form (33000) oder irgendwelche tromboplastinischen Verunreinigungen von u-PA aus dem Serum oder dem Urin zu binden
d) er bindet das aminoterminale Fragment mit einem MG von 18000 der A-Kette
e) er verschlechtert die enzymatische Aktivität von u-PA nicht
f) er hemmt nicht die enzymatische Aktivierung von pro-uPA
g) er besitzt einen isoelektrischen Punkt von etwa 5,75

7. Verfahren nach Anspruch 1, dadurch **gekennzeichnet,** daß der enzymatische Aktivator aus Plasmin, Thrombin, Kallicrein und Trypsin ausgewählt wird.

8. Verfahren nach Anspruch 1, dadurch **gekennzeichnet,** daß bei der amidolytischen Reaktion ein farbentwickelndes Substrat für u-PA verwendet wird.

9. Verfahren nach Anspruch 1, dadurch **gekennzeichnet,** daß bei der amidolytischen Reaktion ein farbentwickelndes Substrat für u-PA verwendet wird, welches Pyro-Glutamyl-Glycyl-Arginyl-p-nitroanilid ist.

10. Kit zur Anwendung bei dem Verfahren nach Anspruch 1, dadurch **gekennzeichnet,** daß er
    a) einen analytischen Träger, der auf der Oberfläche einen monoklonalen Antikörper oder ein übliches Antiserum trägt, welches ein gemeinsames Epitop auf u-PA und pro-uPA erkennt und weder die Aktivität von u-PA hemmt noch die enzymatische Aktivierung von pro-uPA hemmt
    b) einen enzymatischen Aktivator von pro-uPA und gegebenenfalls
    c) ein chromogenes Substrat für u-PA
    umfaßt.

## Revendications

1. Procédé de dosage d'un analyte choisi parmi l'activateur du plasminogène urinaire humain (u-PA) et l'un de ses zymogènes (précurseur de l'activateur de plasminogène urinaire humain, pro-uPA) ou un de leurs mélanges, qui consiste :
    a) à mettre une solution d'essai en contact avec un support analytique portant sur sa surface un anticorps monoclonal ou un antisérum classique qui reconnaît un épitope commun sur l'u-PA et le pro-uPA sans inhiber l'activité de l'u-PA ni inhiber l'activation enzymatique du pro-uPA,
    b) après rinçage, à ajouter une solution tamponnée d'un activateur enzymatique de l'u-PA à une série de supports analytiques traités, tout en n'ajoutant que la solution tamponnée correspondante à une autre série de supports analytiques traités en parallèle,
    c) à déterminer la concentration des analytes selon un dosage amidolytique connu en soi sur un substrat synthétique de développement des couleurs, par référence à une courbe étalon ; la concentration de l'u-PA étant déterminée directement à partir des échantillons qui n'ont pas été traités par l'activateur ezymatique dans l'étape précédente, tandis que la concentration du pro-uPA est déterminée par la différence entre la concentration des analytes totaux, mesurée dans les échantillons traités par l'activateur enzymatique, et les échantillons traités simplement par le tampon, au cours de l'étape précédente.

2. Procédé selon la revendication 1, dans lequel le support analytique est constitué d'une matière plastique.

3. Procédé selon la revendication 1, dans lequel le support analytique est choisi parmi des plaques de microtitrage, tubes à essai ou feuilles en matière plastique, ou des membranes en nitrocellulose ou en Nylon.

4. Procédé selon la revendication 1, dans lequel le support analytique est une plaque de microtitrage en polystyrène, poly(chlorure de vinyle) ou polyéthylène.

5. Procédé selon la revendication 1, dans lequel le support analytique a été revêtu d'un anticorps monoclonal ou d'un anti-sérum classique ayant les caractéristiques suivantes :
    a) il reconnait un épitope commun sur u-PA et pro-uPA et n'inhibe ni l'activité de l'u-PA, ni l'activation enzymatique du pro-uPA ;

b) il a une affinité pour l'antigène comprise entre $10^{-6}$ et $10^{-11}$ ;

c) il se fixe à l'u-PA et à l'un de ses précurseurs à chaîne unique (comme un pro-uPA) ;

d) il ne subit pas de réaction immunologiquement croisée avec les endopeptidases enzymatiquement actives du sérum ou de l'urine, autres que l'u-PA.

**6.** Procédé selon la revendication 1, dans lequel le support analytique a été revêtu d'un anticorps monoclonal ayant les caractéristiques suivantes :

a) il appartient à la classe des $IgG_1$ ;

b) il a une constante d'affinité d'environ $1,42 \times 10^7$ l.mole$^{-1}$ (matrice portant l'urokinase antigène) mesurée essentiellement par le mode opératoire décrit par Tsapis et al. dans Eur. J. Biochem. 64, 369 (1976) ;

c) il se lie d'une manière spécifique à la forme à masse moléculaire élevée (54.000) de l'u-PA et à l'un de ses précurseurs (comme le pro-uPA), sans fixer la forme à faible masse moléculaire (33.000) ou tout contaminant thromboplastinique sérique ou urinaire de l'u-PA ;

d) il fixe le fragment amino-terminal 18.000 Mr de la chaîne A;

e) il ne dégrade pas l'activité enzymatique de l'u-PA ;

f) il n'inhibe pas l'activation enzymatique du pro-uPA ;

g) il a un point isoélectrique d'environ 5,75.

**7.** Procédé selon la revendication 1, dans lequel l'activateur enzymatique est choisi parmi la plasmine, la thrombine, la kallicréine et la trypsine.

**8.** Procédé selon la revendication 1, dans lequel la réaction amidolytique utilise un substrat de développement de couleur pour l'u-PA.

**9.** Procédé selon la revendication 1, dans lequel la réaction amidolytique utilise un substrat de développement de couleur pour l'u-PA, qui est le pyro-glutamyl-glycyl-arginyl-p-nitroanilide.

**10.** Coffret destiné à être utilisé dans le procédé selon la revendication 1, qui comprend :

a) un support analytique portant sur sa surface un anticorps monoclonal ou un anti-sérum classique qui reconnaît un épitope courant de l'u-PA et du pro-uPA, et n'inhibe ni l'activité de l'uPA, ni l'activation enzymatique du pro-uPA,

b) un activateur enzymatique du pro-uPA et, facultativement,

c) un substrat chromogène pour l'u-PA.